# EUROPEAN PATENT APPLICATION

(11) **EP 4 512 809 A1**
(43) Date of publication of application: **26.02.2025**
(21) Application number: 23791952.7
(22) Date of filing: 21.04.2023
(51) Int. Cl.: C07D 487/04, A61K 31/519, A61P 35/00, A61P 35/02

(54) **METHOD FOR PRODUCING 7H-PYRROLO[2,3-D]PYRIMIDINE-4-AMINE DERIVATIVE**

(30) Priority: 22.04.2022 JP 2022071155
(71) Applicant: Taiho Pharmaceutical Co., Ltd., Chiyoda-ku, Tokyo 101-8444 (JP)
(72) Inventor: TAKEDA, Daisuke, Kodama-gun, Saitama 367-0241 (JP); NONOSHITA, Katsumasa, Kodama-gun, Saitama 367-0241 (JP); KOBAYAKAWA, Yu, Tsukuba-shi, Ibaraki 300-2611 (JP)
(74) Representative: Wächtershäuser & Hartz Patentanwaltspartnerschaft mbB
(86) International application number: PCT/JP2023/015917
(87) International publication number: WO 2023/204304

(57) **Abstract**

The present invention provides a method for producing a compound represented by formula (I) suitable for mass synthesis as API. Provided according to one aspect of the present invention is a method for producing a compound represented by formula (I) or a salt thereof, the method including deprotecting a protecting group represented by R₁ in formula (II) under basic conditions to thereby form a compound represented by formula (I) or a salt thereof.

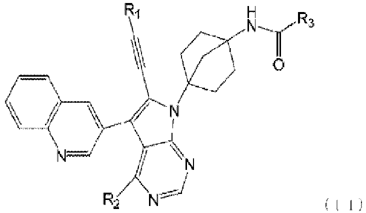

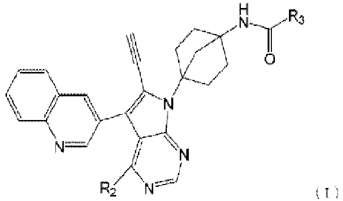

## Description

### TECHNICAL FIELD

The present invention relates to a method for producing a 7H-pyrrolo[2,3-d]pyrimidin-4-amine derivative, and to a 7H-pyrrolo[2,3-d]pyrimidin-4-amine derivative, and the like, which can be mass produced.

### BACKGROUND ART

When an active pharmaceutical ingredient (API) is produced industrially, the process for producing the API from raw ingredients requires few steps and high yield. Furthermore, since production of APIs requires that they be able to be mass produced, operations that are not suitable for mass production are not desirable. The quality of the API produced by such a process must meet the standards, etc. set by the International Council for Harmonisation of Technical Requirements for Pharmaceuticals for Human Use (hereinafter referred to as ICH), where the standards include those for impurities, residual solvents, and residual metal contents in the API.

Patent literature 1 discloses N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (hereinafter may be referred to as compound (1)) as a compound having EGFR inhibitory activity. In addition, as an exemplary method for producing the compound, it discloses a route for synthesizing a compound represented by formula (XXVI), in which a compound represented by formula (XXII) is reacted with a compound represented by formula (XXIII) through Sonogashira coupling to introduce a R₁-C≡C- group (R₁ is a hydrogen atom or an optionally substituted C1-C3 alkyl) to obtain a compound represented by formula (XXIV), which is then converted into formula (XXV) by amine deprotection, and finally subjected to acylation reaction.

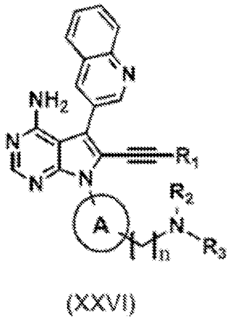

### CITATION LIST

### Patent Literature

Patent literature 1: WO2020/166680

### SUMMARY OF THE INVENTION

### PROBLEM TO BE SOLVED BY THE INVENTION

However, there was room for further investigation of a method suitable for mass production as a synthesis route for the compound. Under these circumstances, there is a need for a method for producing compound (1), or a compound represented by formula (I) described below, which is suitable for mass synthesis as an API. In particular, there is a need for a method for producing compound (1), or a compound represented by formula (I) described below, which is superior in one or more characteristics from low impurity content and high yield among the characteristics suitable for mass synthesis as an API.

### MEANS TO SOLVE THE PROBLEM

The present inventors, after diligent research, have completed a method for producing a compound represented by formula (I) having one or more characteristics suitable for mass synthesis by deprotecting a protecting group of a terminal acetylene under basic conditions.

Thus, the present invention provides, for example, the following [1]-[14].
[1] A method for producing a compound represented by formula (I) or a salt thereof, the method comprising deprotecting a protecting group represented by R₁ in formula (II): 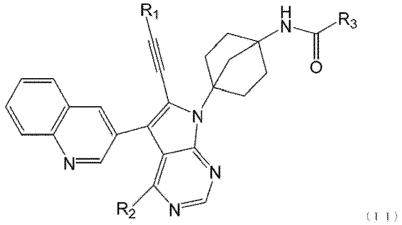 under basic conditions to form a compound represented by formula (I) or a salt thereof: 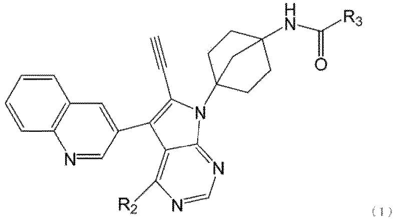
   wherein, in formulae (I) and (II) above,
   R₁ represents a protecting group,
   R₂ represents an optionally protected amino (amine), a halogen atom, or a leaving group, and
   R₃ represents an optionally substituted 5-10 membered monocyclic or polycyclic unsaturated heterocyclic ring group.
[2] The method according to [1], wherein, in formulae (I) and (II), R₁ represents a silicon-linked protecting group, R₂ represents an amino (amine), and R₃ represents a pyrazinyl optionally having a methyl group.
[3] The method according to [1] or [2], wherein the compound represented by formula (I) is N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof.
[4] A method for producing a compound represented by formula (I) or a salt thereof, the method comprising:
   (1) linking a compound represented by formula (III-1) with a compound represented by formula (VIII) to give a compound represented by formula (III-2), or linking a compound represented by formula (III-1) with a compound represented by formula (IV) to produce a compound represented by formula (V-1): 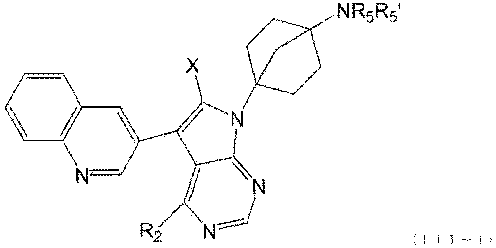 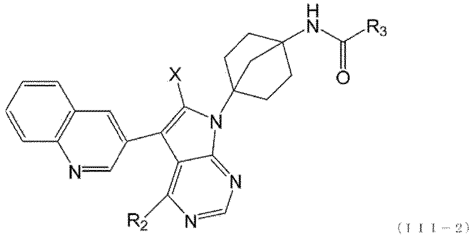 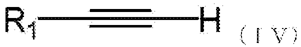 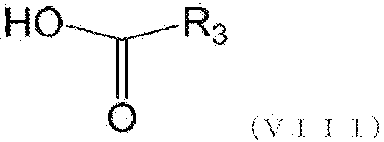 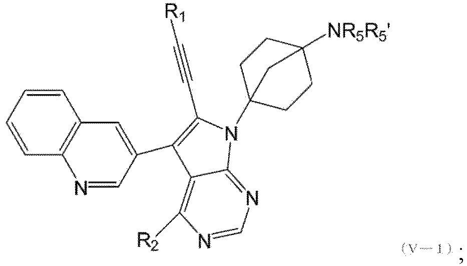
   (2) linking the compound represented by formula (III-2) with the compound represented by formula (IV) or linking the compound represented by formula (V-1) with the compound represented by formula (VIII) to give a compound represented by formula (II); and 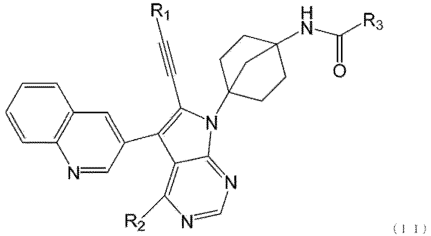
   (3) deprotecting a protecting group represented by R₁ in formula (II) under basic conditions to form the compound represented by formula (I) or a salt thereof:

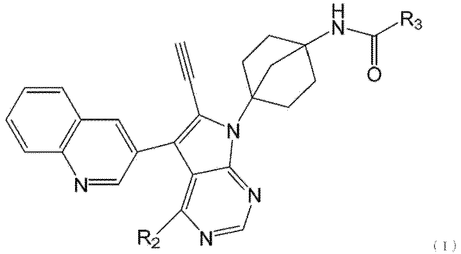

   wherein, in formulae (I), (II), (III-1), (III-2), (IV), (V-1), and (VIII) above,
   R₁ represents a protecting group,
   R₂ represents an optionally protected amino (amine), a halogen atom, or a leaving group,
   R₃ represents an optionally substituted 5-10 membered monocyclic or polycyclic unsaturated heterocyclic ring group,
   R₅ and R₅' each independently represent a hydrogen atom or a protecting group of an amino (amine), or NR₅R₅' represents a phthalimide or a nitro, and
   X represents a halogen atom or a leaving group.
[5] The method according to [4], wherein in formulae (I), (II), (III-1), (III-2), (IV), and (V-1) above,
   R₁ represents a silicon-linked protecting group,
   R₂ represents an amino (amine),
   R₃ represents a pyrazinyl optionally having a methyl group, and
   R₅ represents a hydrogen atom or Boc, and R₅' represents a hydrogen atom.
[6] The method according to [4] or [5], wherein the compound represented by formula (I) is N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof.
[7] The method according to [4], further comprising:
   reacting a compound represented by formula (VI): 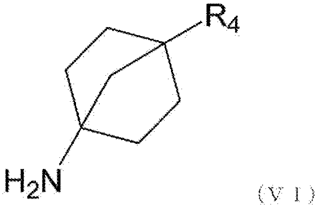 with a compound represented by formula (VII): 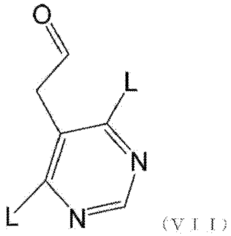
   wherein, L represents a halogen atom or a leaving group, to obtain a compound of formula (IX): 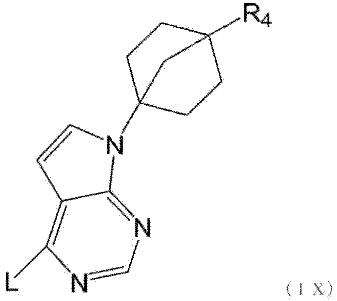
   wherein, in formula (VI) and formula (IX),
   R₄ represents NR₅R₅',
   R₅ and R₅' each independently represent a hydrogen atom or a protecting group of an amino (amine), or NR₅R₅' represents a phthalimide or a nitro, and
   L represents a halogen atom or a leaving group; and
   obtaining a compound represented by formula (III-1) from the compound of formula (IX).
[8] The method according to [7], wherein in formulae (VI), (VII), and (IX) above,
   R₄ represents NR₅R₅',
   R₅ represents Boc, and R₅' represents a hydrogen atom, and
   L represents a chlorine atom.
[9] The method according to any one of [4]-[8], wherein the method does not comprise purification by column chromatography.
[10] The method according to any one of [1]-[9], wherein the content of a compound represented by formula (I-1): 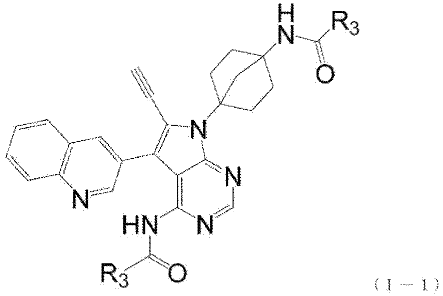 wherein, R₃ represents an optionally substituted 5-10 membered monocyclic or polycyclic unsaturated heterocyclic ring group, in the compound represented by formula (I) and a salt thereof produced by the method is 0.2% or less.
[11] A composition obtained by the method according to any one of [1]-[10], comprising the compound represented by formula (I) or a salt thereof.
[12] A compound represented by formula (II) or a salt thereof: 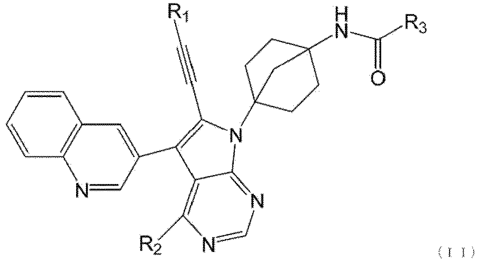 wherein,
   R₁ represents a protecting group,
   R₂ represents an optionally protected amino (amine), a halogen atom, or a leaving group, and
   R₃ represents an optionally substituted 5-10 membered monocyclic or polycyclic unsaturated heterocyclic ring group.
[13] The compound or a salt thereof according to [12], wherein, in formula (II), R₁ represents a silicon-linked protecting group, R₂ represents an amino (amine), and R₃ represents a pyrazinyl optionally having a methyl group.
[14] The compound or a salt thereof according to [12] or [13], wherein the compound represented by formula (II) is N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide or a salt thereof.

### EFFECTS OF THE INVENTION

A method according to one aspect of the present invention can provide a method for producing compound (1), or a compound represented by formula (I), which is superior in one or more characteristics from low impurity content and high yield among the characteristics suitable for mass synthesis as an API. A method according to a preferred aspect of the present invention can provide a method having one or more of the following additional characteristics: no fluorine-containing reagent is used, no explosive reagent is used, and purification by column chromatography can be avoided.

According to one aspect of the present invention, there is provided a method for producing a compound represented by formula (I) or a salt thereof without purification by column chromatography, the method comprising deprotecting a protecting group represented by R₁ of a compound represented by formula (II) under basic conditions. On the other hand, the method described in Patent literature 1 may require purification by silica gel column chromatography.

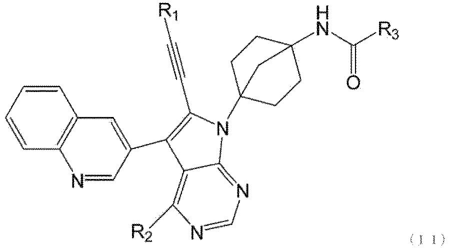

Wherein, R₁ represents a protecting group, R₂ represents an optionally protected amino, a halogen atom, or a leaving group, and R₃ represents an optionally substituted 5-10 membered monocyclic or polycyclic unsaturated heterocyclic ring group.

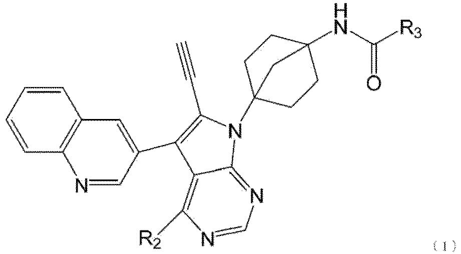

Wherein, R₂ and R₃ are the same as above.

The step of converting formula (II) into formula (I) will be described below.

Herein, R₁ represents a protecting group, which is a substituent for protecting the hydrogen atom of the terminal acetylene in formula (I). A terminal acetylene is an acetylene in which one of the two hydrogen atoms in the acetylene has been replaced, and refers to -C≡CH (also called an ethynyl) in formula (I). In an embodiment of the present invention, any protecting group can be used as R₁ as long as it is a protecting group that can be deprotected under basic conditions. Examples of such a protecting group in R₁ include a protecting group that replaces the hydrogen atom of the terminal acetylene with a carbon atom (carbon-linked protecting group), a protecting group that replaces the hydrogen atom with a silicon atom (silicon-linked protecting group), and a protecting group that replaces the hydrogen atom with a phosphorus atom (phosphorus-linked protecting group).

Examples of the carbon-linked protecting group herein include a tert-butyl, a cyclopropyl, a 2-hydroxypropan-2-yl, and a dimethylaminomethyl, preferably 2-hydroxypropan-2-yl.

Examples of the silicon-linked protecting group herein include TMS (trimethylsilyl), TES (triethylsilyl), TBS (tert-butyldimethylsilyl), TIPS (triisopropylsilyl), and TBDPS (tert-butyldiphenylsilyl). It is preferably TES or TIPS, and more preferably TES.

Examples of the phosphorus-linked protecting group herein include -PPh₂ (diphenylphosphine) and -P(O)Ph₂, preferably -P(O)Ph₂ (Ph represents a phenyl).

Since R₁ in an embodiment of the present invention is a protecting group that can be deprotected under basic conditions, an analog such as a compound represented by formula (I-1), which may possibly be generated as a by-product during the reaction step, can be converted into a target compound simultaneously with deprotection. Through this conversion, impurity content can be reduced and a high yield can be realized, thereby achieving a high degree of purity as a raw ingredient for pharmaceuticals.

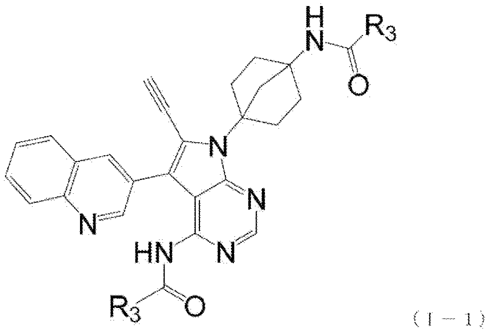

Wherein, R₃ is the same as above.

In an embodiment of the present invention, R₁ is preferably a protecting group selected from the group consisting of carbon-linked protecting groups, silicon-linked protecting groups, and phosphorus-linked protecting groups, more preferably a silicon-linked protecting group, more preferably TMS (trimethylsilyl), TES (triethylsilyl), TBS (tert-butyldimethylsilyl), TIPS (triisopropylsilyl), or TBDPS (tert-butyldiphenylsilyl), and particularly preferably TES (triethylsilyl).

R₂ of the compound used in the method according to an embodiment of the present invention is an optionally protected amino, a halogen atom, or a leaving group.

Examples of the protecting group of the "optionally protected amino" in R₂ include, but is not limited to, carbamate protecting groups such as a tert-butoxycarbonyl (Boc), a benzyloxycarbonyl (Cbz or Z), a 9-fluorenylmethyloxycarbonyl (Fmoc), an allyloxycarbonyl (Alloc), and a 2,2,2-triethoxycarbonyl (Troc); sulfonamide protecting groups such as an o-nitrobenzenesulfonyl (o-Ns, Ns, nosyl, or o-nosyl), a p-nitrobenzenesulfonyl (p-Ns or p-nosyl), a 2,4-dinitrobenzenesulfonyl (DNs), and SES (2-trimethylsilylethanesulfonyl); and protecting groups where R₂ is a phthaloyl (Phth), and it is preferably an amino, an amino with a carbamate protecting group, or a phthalimide, more preferably an amino or an amino having one or two of a protecting group selected from the group consisting of Boc, Cbz, Fmoc, Alloc, and Troc, more preferably an amino or an amino having one or two of a protecting group selected from the group consisting of Boc, Cbz, and Fmoc, and particularly preferably an amino.

Examples of the halogen atom in R₂ include a fluorine atom, a chlorine atom, a bromine atom, and an iodine atom, and it is preferably a chlorine atom.

Examples of the leaving group in R₂ include -OSO₂CₙFₙ₊₂ (n represents an integer from 1-4), -SO₂CₙFₙ₊₂, a mesylate (-OMs; Ms represents a mesyl), a mesyl, a tosylate (-OTs; Ts represents a p-tosyl), a p-tosyl, a nosylate (-ONs or -Op-Ns), a p-nosyl, -OSO₂Ph (Ph represents a phenyl), -SO₂Ph, and a phenoxy (-OPh), and it is preferably a mesyl. Alternatively, the leaving group may be -OSO₂CₙF₂ₙ₊₁ (n represents an integer from 1-4), -SO₂CₙF₂ₙ₊₁, a mesylate (-OMs; Ms represents a mesyl), a mesyl, a tosylate (-OTs; Ts represents a p-tosyl), a p-tosyl, a nosylate (-ONs or -Op-Ns), a p-nosyl, -OSO₂Ph (Ph represents a phenyl), -SO₂Ph, a phenoxy (-OPh), or the like.

R₂ of the compound used in the method according to an embodiment of the present invention is an optionally protected amino, a halogen atom, or a leaving group, or R₂ is a phthalimide. It is preferably an amino, an amino having one or two of a protecting group selected from the group consisting of Boc, Cbz, Fmoc, Alloc, Troc, and Ns, a fluorine atom, a chlorine atom, a bromine atom, an iodine atom, -OSO₂CₙFₙ₊₂ (n represents an integer from 1-4), -SO₂CₙFₙ₊₂, a mesylate, a mesyl, a tosylate, a p-tosyl, a nosylate, a p-nosyl, -OSO₂Ph, -SO₂Ph, or a phenoxy, or R₂ is a phthalimide, preferably an amino, Boc, Cbz, Fmoc, a chlorine atom, or a mesyl, more preferably an amino, a chlorine atom, or a mesyl, and particularly preferably an amino. Alternatively, R₂ may be an amino, an amino having one or two of a protecting group selected from the group consisting of Boc, Cbz, Fmoc, Alloc, Troc, and Ns, a fluorine atom, a chlorine atom, a bromine atom, an iodine atom, -OSO₂CₙF₂ₙ₊₁ (n represents an integer from 1-4), -SO₂CₙF₂ₙ₊₁, a mesylate, a mesyl, a tosylate, a p-tosyl, a nosylate, a p-nosyl, -OSO₂Ph, -SO₂Ph, or a phenoxy, or R₂ may be a phthalimide.

In an embodiment of the present invention, R₃ of the compound represented by formula (I) or formula (II) is an optionally substituted 5-10 membered monocyclic or polycyclic unsaturated heterocyclic group. "An optionally substituted 5-10 membered monocyclic or polycyclic unsaturated heterocyclic group" is preferably a 5-10 membered, monocyclic or polycyclic, fully unsaturated or partially saturated heterocyclic group having 1-4 heteroatoms selected from nitrogen, oxygen and sulfur atoms, more preferably a pyridazinyl, a pyrimidinyl, an oxazolyl, an oxadiazolyl, a pyrazinyl, a pyridinyl, an imidazolyl, a furanyl, an isoxazolyl, a triazolopyridinyl, a triazolyl, a triazinyl, a thiazolyl, a thiadiazolyl, an imidazopyrazinyl, or a pyrazolyl, still more preferably an isoxazolyl, a pyrazolyl, an oxazolyl, an oxadiazolyl, a pyrazinyl, or a pyridazinyl, yet still more preferably a pyrazolyl, an oxazolyl, an oxadiazolyl, a pyrazinyl, or a pyridazinyl, and particularly preferably a pyrazinyl.

In an embodiment of the present invention, the "substituent" of "an optionally substituted 5-10 membered monocyclic or polycyclic unsaturated heterocyclic group" represented by R₃ is a halogen atom, a cyano, an oxo, a C1-C6 alkyl, a C1-C6 haloalkyl, a C1-C6 alkoxy-C1-C6 alkyl, a C1-C6 alkoxy, a C1-C6 haloalkoxy, a C1-C6 mono- or di-alkylamino, a C1-C6 alkylsulfonyl, a C3-C7 cycloalkyl, or a C6-C10 aromatic hydrocarbon. It is preferably a fluorine atom, a chlorine atom, a cyano, an oxo, a C1-C6 alkyl, a monofluoromethyl, a methoxyethyl, a methoxy, a monofluoromethoxy, a dimethylamino, a methylsulfonyl, a cyclopropyl, or a phenyl, more preferably a C1-C6 alkyl, and still more preferably a methyl. Herein, the number of substituents may be one or more.

In an embodiment of the present invention, while R₃ is an optionally substituted 5-10 membered monocyclic or polycyclic unsaturated heterocyclic group, it is preferably an optionally substituted, 5-10 membered, monocyclic or polycyclic, fully unsaturated or partially saturated heterocyclic group having 1-4 heteroatoms selected from nitrogen, oxygen and sulfur atoms, more preferably an optionally substituted pyridazinyl, pyrimidinyl, oxazolyl, oxadiazolyl, pyrazinyl, pyridinyl, imidazolyl, furanyl, isoxazolyl, triazolopyridinyl, triazolyl, triazinyl, thiazolyl, thiadiazolyl imidazopyrazinyl, or pyrazolyl, more preferably an optionally substituted pyrazinyl, more preferably a pyrazinyl which may have a C1-C6 alkyl, more preferably a pyrazinyl optionally having a methyl group, and more preferably 5-methylpyrazin-2-yl.

In a preferred embodiment of the present invention, the compound represented by formula (I) is N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide. As shown in Patent literature 1, this compound is preferred because it has excellent inhibitory activity.

In an embodiment of the present invention, a by-product that is generated upon conversion of the compound represented by formula (II) into the compound represented by formula (I) is not particularly limited as long as it is a compound other than the compound represented by formula (I), but it is preferably a by-product that is converted into the compound represented by formula (I) under basic conditions, and more preferably a compound represented by formula (I-1).

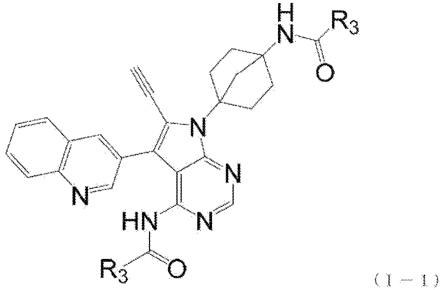

Wherein, in the formula, the definition and embodiment of R₃ are the same as above.

For example, when R₃ is 5-methylpyrazin-2-yl, the compound represented by formula (I-1) is N-(4-(6-ethynyl-4-(5-methylpyrazine-2-carboxamide)-5-(quinolin-3-yl)-7H-pyrrolo[2,3-d]pyrimidin-7-yl)bicyclo[2.2.1]heptan-1-yl)-5-methylpyrazine-2-carboxamide (hereinafter referred to as compound (2)), having the following structure.

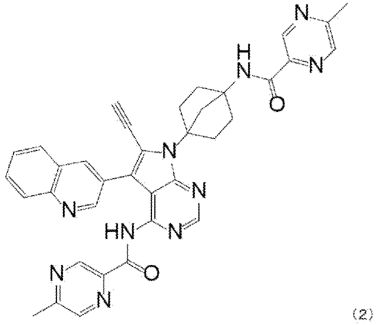

According to the present invention, basic conditions refer to a state in which the reaction system is adjusted to be basic with a base. Examples of this base are not particularly limited as long as the reaction proceeds and include inorganic bases such as metal hydroxides (sodium hydroxide, calcium hydroxide, etc.), metal hydrides (lithium hydride, sodium hydride, etc.), and metal carbonates (sodium carbonate, potassium carbonate, cesium carbonate, calcium carbonate, lithium carbonate, magnesium carbonate, sodium bicarbonate, etc.), and organic bases such as metal alkoxides (sodium methoxide, potassium tert-butoxide, etc.), metal amides (sodium amide, lithium diisopropylamide, etc.), alkyl metal compounds (n-butyllithium, trimethylaluminum, etc.), alkylamines (triethylamine, tetramethylethylenediamine, piperidine, 1,4-diazabicyclo[2.2.2]octane, etc.), heterocyclic amines (diazabicycloundecene, pyridine, imidazole, etc.), and quaternary ammonium fluorides (tetra-n-butylammonium fluoride). It is preferably a base that does not contain a fluoride ion, more preferably a metal carbonate or a salt containing potassium that does not contain a fluoride ion, and more preferably potassium carbonate. These can be used alone or in combination in the method of the present invention.

While the amount of this reagent to be used is not particularly limited as long as the reaction proceeds, it can be used in an amount of, for example, 0.1-50 moles per mole of the compound represented by formula (II). The amount is preferably 0.1-10 moles, and more preferably 0.1-2 moles.

The solvent for the deprotection step is not particularly limited as long as the reaction system can be adjusted to be basic with a base and the compound used in the reaction can be dissolved. Examples include a C5-C10 hydrocarbon, a C6-C14 aromatic hydrocarbon, a C1-C6 alcohol, a C3-C10 aliphatic carboxylate, a C3-C10 ketone, a C4-C10 ether, a non-protic polar organic solvent, or a mixed solvent thereof. Herein, the alphabet C followed by a number represents the number of carbon atoms, indicating the total number of carbons contained in the compound.

The C5-C10 hydrocarbon is a hydrocarbon with 5-10 carbon atoms, such as pentane, hexane, heptane, octane, nonane, decane, cyclopentane, cyclohexane, cycloheptane, cyclooctane, cyclononane, and cyclodecane.

The C6-C14 aromatic hydrocarbon is an aromatic hydrocarbon with 6-14 carbon atoms, such as benzene, naphthalene, anthracene, toluene, xylene, cumene, styrene, and phenanthrene.

The C1-C6 alcohol is an alcohol with 1-6 carbon atoms, such as methanol, ethanol, n-propanol, isopropanol, n-butanol, tert-butanol, n-pentanol, and n-hexanol.

The C3-C10 aliphatic carboxylate is an aliphatic carboxylate with 3-10 carbon atoms, such as propyl formate, methyl acetate, ethyl acetate, propyl acetate, methyl propionate, ethyl propionate, propyl propionate, methyl butanoate, and ethyl butanoate.

The C3-C10 ketone is a ketone with 3 to 10 carbon atoms, such as acetone, ethyl methyl ketone, diethyl ketone, isopropyl methyl ketone, and cyclohexanone.

The C4-C10 ether is an ether with 4-10 carbon atoms, such as diethyl ether, tert-butyl methyl ether, tetrahydrofuran, and 1,4-dioxane.

The non-protic polar organic solvent is a solvent that has no proton to ionize, such as N,N-dimethylformamide, N,N-dimethylacetamide, N-methylpyrrolidone, chloroform, and dichloromethane.

Among the above solvents, dichloromethane, methanol, ethanol, and N-methylpyrrolidone are preferable, and dichloromethane and methanol are more preferable. While the amount of the solvent is not particularly limited as long as the reaction proceeds, it is preferably 10-100 L, more preferably 15-50 L and still more preferably 20-30 L per 1 kg of the compound of formula (II).

The reaction time of the deprotection step is not particularly limited and can be appropriately determined as long as the deprotection can be achieved. For example, the reaction time is 1-120 hours, preferably 6-48 hours, and more preferably 12-24 hours.

The reaction temperature of the deprotection step is not particularly limited and can be appropriately determined as long as the deprotection can be achieved. For example, the reaction temperature is 0-60°C, preferably 10-50°C, and more preferably 20-40°C. (According to the present invention, room temperature refers to 18-25°C).

Another aspect of the present invention provides a compound represented by formula (II). The compound represented by formula (II) is a useful intermediate for producing the compound represented by formula (I). In an embodiment of the present invention, any of the above options for R₁, R₂ and R₃ can be combined as appropriate in the compound represented by formula (II).

### <Reaction pathway>

A method according to an embodiment of the present invention comprises:
(1) reacting a compound represented by formula (III) with a compound represented by formula (IV) to produce a compound represented by formula (V): 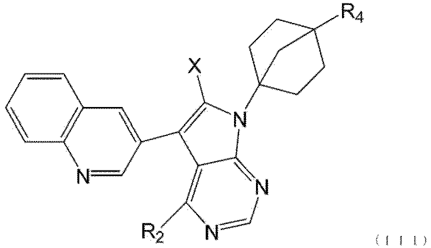 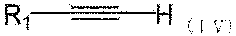 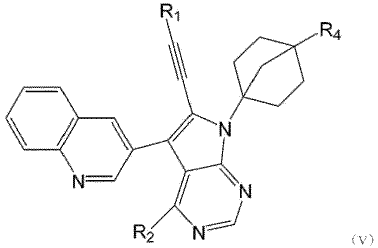
   wherein, in formulae (III)-(V) above,
   R₁ represents a protecting group,
   R₂ represents an optionally protected amino, a halogen atom, or a leaving group, and
   R₃ represents an optionally substituted 5-10 membered monocyclic or polycyclic unsaturated heterocyclic ring group,
   R₄ represents NR₅R₅' or NHCOR₃, wherein R₅ and R₅' each independently represent a hydrogen atom or a protecting group of an amino, or NR₅R₅' represents a phthalimide or a nitro, and
   X represents a halogen atom or a leaving group;
(2) deprotecting the protecting group represented by R₁ in formula (V) under basic conditions; and
(3) optionally, making R₄ of the compound represented by formula (III) to be NHCOR₃ before (1) above, or making R₄ of the compound represented by formula (V) to be NHCOR₃ after (1) and before (2) above. The reaction in (1) above includes a coupling reaction and the reaction in (3) above includes a condensation reaction. According to one preferred embodiment of the present invention, R₄ of the compound represented by formula (V) is made to be NHCOR₃ after (1) and before (2) above.

An embodiment of the present invention provides a method, wherein R₄ of the compound represented by formula (III) above and/or R₄ of the compound represented by formula (V) above is NR₅R₅', at least one of R₅ and R₅' is a protecting group of an amino, and the method further comprises deprotecting the protecting group of the amino. In one preferred embodiment of the present invention, the protecting group of the amino is deprotected before (1) above, or after (1) and before (2) above, preferably deprotected after (1) and before (2) above.

Next, the condensation reaction and Sonogashira coupling, which are performed before the deprotection in an embodiment of the present invention, will be described. The phrase "before the deprotection in an embodiment of the present invention" here applies to cases immediately before said deprotection or cases further before that, as long as it precedes the "deprotection of the protecting group of the terminal acetylene" described above. The order of the "condensation reaction" and the "Sonogashira coupling" here is not questioned, and there may be another reaction step in between the "condensation reaction" and the "Sonogashira coupling".

### (When R₄ is NHCOR₃)

When R₄ is NHCOR₃ in the compound of formula (III) above, the compound represented by formula (I) can be obtained by performing the above steps (1) and (2) using a compound represented by formula (III-2) below as the starting material. In this case, the compound represented by formula (I) can be obtained without performing step (3). In the following reaction formula, "Deprotection" refers to "deprotection".

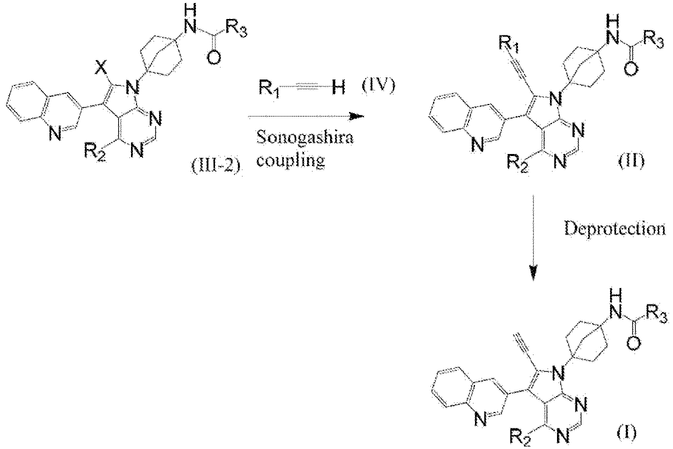

### (When R₄ is NR₅R₅')

In an embodiment of the present invention, when R₄ is NR₅R₅', the compound of formula (I) can be produced by using formula (III-1) below as the starting material to make R₄ of the compound represented by formula (III) to be NHCOR₃ before (1) above or make R₄ of the compound represented by formula (V) to be NHCOR₃ after (1) and before (2) above so that deprotection is finally performed under basic conditions.

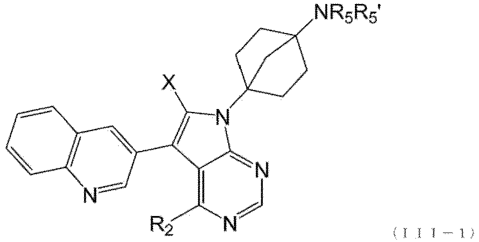

Such a procedure for producing a compound of formula (I) (synthesis route 1) is shown below. In synthesis route 1, "Condense" refers to "condensation reaction" and "Deprotection" refers to "deprotection".

### Synthesis route 1

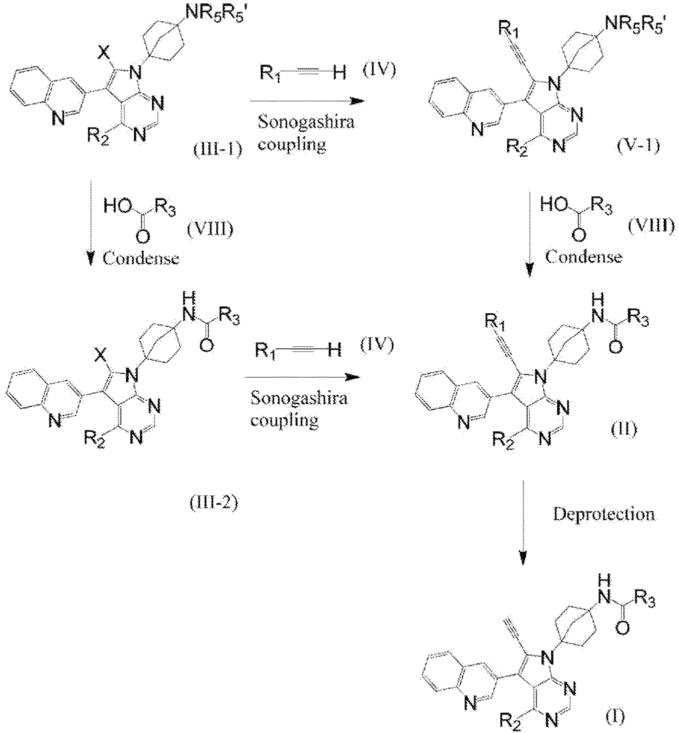

In an embodiment of the present invention, R₅ and R₅' each independently represent a hydrogen atom or a protecting group of an amino, or NR₅R₅' represents a phthalimide or a nitro.

When R₅ and/or R₅' is a protecting group of an amino or NR₅R₅' is a phthalimide, conversion of the compound represented by formula (III-1) into the compound represented by formula (III-2) and conversion of the compound represented by formula (V-1) into the compound represented by formula (II) in synthesis route 1 include the step of deprotecting R₅ and/or R₅'.

The "protecting group" in R₅ and R₅' may be the same protecting group as those listed for R₂, preferably a carbamate protecting group, and more preferably Boc.

According to the present invention, R₅ and R₅' each independently represent a hydrogen atom or a protecting group, or NR₅R₅' represents a phthalimide or a nitro, preferably R₅ and R₅' independently represent a hydrogen atom or a protecting group, more preferably at least one of R₅ and R₅' represents a protecting group, more preferably at least one of R₅ and R₅' represents Boc, and particularly preferably R₅ is a hydrogen atom or Boc and R₅' is a hydrogen atom.

When NR₅R₅' is a nitro, conversion of the compound represented by formula (III-1) into the compound represented by formula (III-2) and conversion of the compound represented by formula (V-1) into the compound represented by formula (II) in synthesis route 1 include the step of reducing the nitro to an amino. A generally known method can be used for such a reduction, and, specifically, the method described in the section "Condensation reaction" below can be used.

Since this synthesis route avoids the use of Ohira-Bestmann method, Sonogashira coupling, condensation reaction (Condense), and conversion of formula (II) into formula (I) can be carried out in any order, but it is preferable to produce the compound represented by formula (I) in the following order (A)-(C) from the viewpoints that the use of column chromatography can be avoided, the yield is high, and the content of impurities can be reduced compared to the method described in Patent literature 1.
(A) Conversion into a compound represented by formula (III-2) by linking a compound represented by formula (III-1) with a compound represented by formula (VIII), or conversion into a compound represented by formula (V-1) by linking a compound represented by formula (III-1) with a compound represented by formula (IV).
(B) Conversion into a compound represented by formula (II) by linking the compound represented by formula (III-2) with a compound represented by formula (IV) or linking the compound represented by formula (V-1) with a compound represented by formula (VIII).
(C) Conversion of the compound represented by formula (II) into the compound represented by formula (I) under basic conditions.

For example, when X is a bromine atom, R₁ is TES, R₂ is an amino, R₃ is 5-methylpyrazin-2-yl, and NR₅R₅' is a Boc-protected amino (e.g. NHBoc, i.e. one of R₅ and R₅' is a hydrogen atom and the other is a protecting group of the amino), compound (1) can be prepared from tert-butyl(4-(4-amino-6-bromo-5-(quinolin-3-yl)-7H-pyrrolo[2,3-d]pyrimidin-7-yl)bicyclo[2,2,1]heptan-1-yl)carbamate by performing Sonogashira coupling, deprotection of Boc (de Boc), and condensation reaction (Condense) in any order, followed by deprotection of TES (de TES).

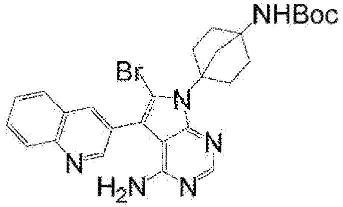

Such a synthesis procedure (synthesis route 2) is shown below.

### Synthesis route 2

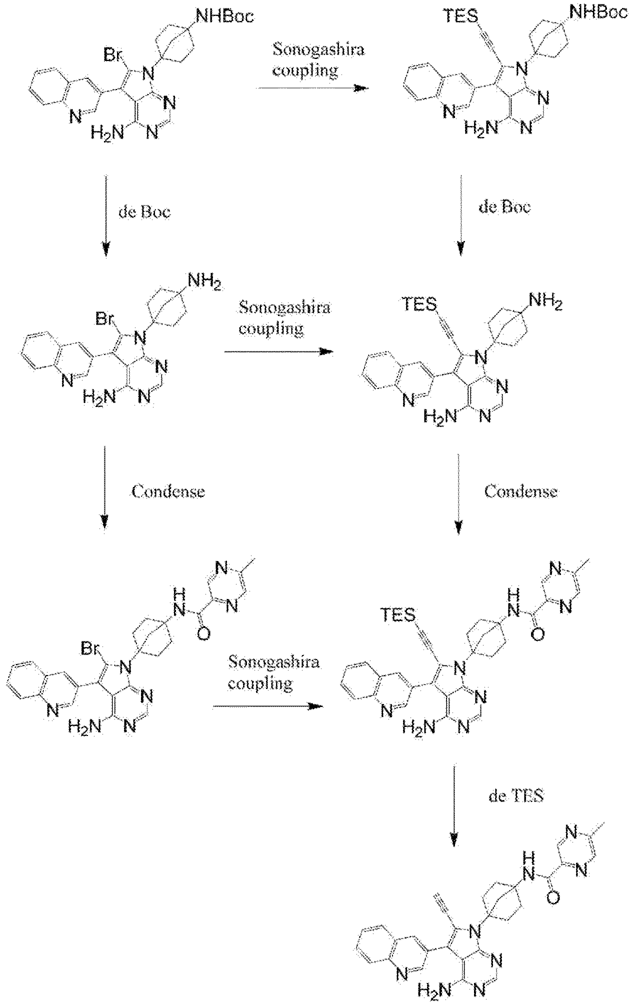

Since this synthesis route avoids the use of Ohira-Bestmann method, Sonogashira coupling, deprotection of Boc (de Boc), condensation reaction (Condense), and deprotection of TES (de TES) can be carried out in any order, but it is preferable to produce compound (1) in the following order (A)-(D) from the viewpoints that the use of column chromatography can be avoided, the yield is high, and the content of impurities can be reduced compared to the method described in Patent literature 1.
(A) Coupling of tert-butyl(4-(4-amino-6-bromo-5-(quinolin-3-yl)-7H-pyrrolo[2,3-d]pyrimidin-7-yl)bicyclo[2,2,1]heptan-1-yl)carbamate with triethylsilylacetylene.
(B) Deprotection of Boc of the compound obtained in (A).
(C) Condensation of the compound obtained in (B) with 5-methylpyrazine-2-carboxylic acid.
(D) Deprotection of TES of the compound obtained in (C) under basic conditions.

More preferably, compound (1) is produced in the following order (A)-(D).
(A) Coupling of tert-butyl(4-(4-amino-6-bromo-5-(quinolin-3-yl)-7H-pyrrolo[2,3-d]pyrimidin-7-yl)bicyclo[2,2,1]heptan-1-yl)carbamate with triethylsilylacetylene using a palladium catalyst and a copper catalyst.
(B) Deprotection of Boc of the compound obtained in (A).
(C) Condensation of the compound obtained in (B) with 5-methylpyrazine-2-carboxylic acid using a combination of EDC and HOBt.
(D) Deprotection of TES of the compound obtained in (C) under basic conditions adjusted by a reagent containing no fluoride ion.

Thus, when the compound of formula (I) or a salt thereof is obtained in this way, the content of impurities represented by formula (I-1) is low. The impurity content can be determined as the percentage (%) of the peak area obtained at a specific wavelength by measurements by HPLC (high performance liquid chromatography) or UPLC (ultra-high performance liquid chromatography). The wavelength can be set appropriately according to the characteristics of the compound. This is important to maintain the quality of pharmaceutical products, and the content of impurities represented by formula (I-1) may be 0.2% or less considering the standards, etc. set by the International Council for Harmonisation of Technical Requirements for Pharmaceuticals for Human Use (ICH). The content is preferably 0.15% or less, more preferably 0.1% or less, and still more preferably 0.05% or less.

In the compound represented by formula (I) above and a salt thereof produced by the method in an embodiment of the present invention, the content of the compound represented by formula (I-1): 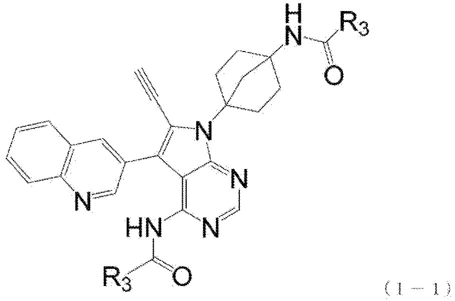 wherein, R₃ represents an optionally substituted 5-10 membered monocyclic or polycyclic unsaturated heterocyclic ring group,
is 0.2% or less, preferably 0.15% or less, more preferably 0.1% or less, and still more preferably 0.05% or less. The compound represented by formula (I-1) does not have to be produced by the method according to an embodiment of the present invention, and thus the above percentage may be below the detection limit.

According to one aspect of the present invention, there is provided a composition obtained by the method described above, comprising a compound represented by formula (I) or a salt thereof. The composition in an embodiment of the present invention comprises a compound represented by formula (I) or a salt thereof and a compound represented by formula (I-1). In a preferred embodiment, the percentage of the compound represented by formula (I-1) in the composition is 0.2% or less, preferably 0.15% or less, more preferably 0.1% or less, and still more preferably 0.05% or less.

Impurities generated by this method can be detected by a known method using known equipment. For example, HPLC can be used for the detection. This HPLC may be a common commercially available HPLC.

In the present invention, the compound represented by formula (I-1) or a salt thereof can be used as a standard product for controlling stability of a formulation or API containing the compound (1) or a salt thereof and controlling quality by confirming the presence of various analogs by the measurement of the infrared absorption (IR) spectrum, nuclear magnetic resonance (NMR) spectrum, and chromatography such as HPLC and thin layer chromatography (TLC) in the method. The compound represented by formula (I-1) is preferably N-(4-(6-ethynyl-4-(5-methylpyrazine-2-carboxamide)-5-(quinolin-3-yl)-7H-pyrrolo[2,3-d]pyrimidin-7-yl)bicyclo[2.2.1]heptan-1-yl)-5-methylpyrazine-2-carboxamide.

### (Condensation reaction)

The condensation reaction performed in the present invention is a step of condensing an amine of a compound represented by formula (III-1) or formula (V-1) below with a carboxylic acid represented by formula (VIII) below to give a compound represented by formula (III-2) or a compound represented by formula (II) having an amide. In other words, this condensation reaction is performed for the construction of an amide bond represented by -NHCOR₃ of the compound represented by formula (III-2) or the compound represented by formula (II). 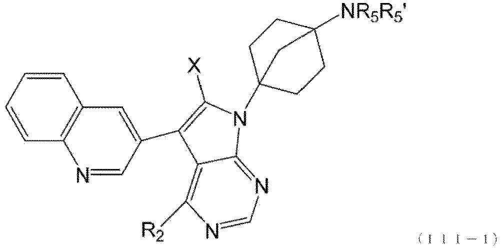 Wherein, X represents a halogen atom or a leaving group, and the definitions and embodiments of R₂, R₅ and R₅' are the same as above. 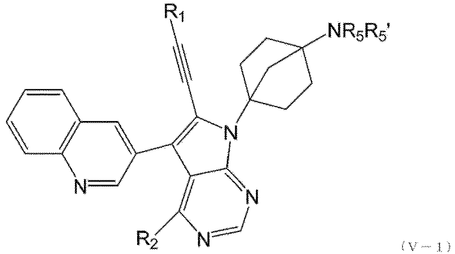 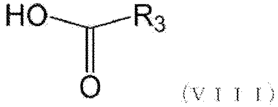 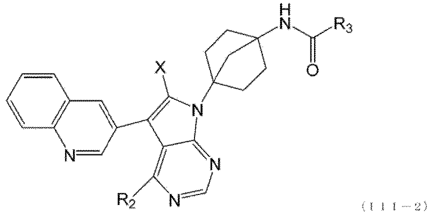 Wherein, in each of the formulae above, X represents a halogen atom or a leaving group, and the definitions and embodiments of R₁, R₂, R₃, R₅ and R₅' are the same as above.

Herein, X of the compound represented by formula (III-1) or formula (III-2) is a halogen atom or a leaving group. Examples of the "halogen atom" include a fluorine atom, a chlorine atom, a bromine atom, and an iodine atom, preferably a chlorine atom, a bromine atom, and an iodine atom, more preferably a bromine atom and an iodine atom, and particularly preferably a bromine atom.

Examples of the "leaving group" include the above leaving groups, preferably - OSO₂CₙFₙ₊₂ (n represents an integer from 1-4), a mesylate, a tosylate, a nosylate, and - OSO₂Ph. Alternatively, the leaving group is preferably -OSO₂CₙF₂ₙ₊₁ (n represents an integer from 1-4), a mesylate, a tosylate, a nosylate, and -OSO₂Ph.

When R₅ and R₅' are hydrogen atoms in the compound represented by formula (III-1) or formula (V-1) used in this condensation reaction, an amide can be constructed by condensation with formula (VIII) by a method described below.

In this condensation reaction, the amount of compound represented by formula (VIII) per mole of the compound represented by formula (III-1) or formula (V-1) is, for example, 0.5-5.0 moles, preferably 0.7-2.5 moles, more preferably 0.8-1.5 moles, still more preferably 0.9-1.3, and particularly preferably 0.9-1.1 moles.

When at least one of R₅ and R₅' represents a protecting group of an amino or NR₅R₅' represents a phthalimide in the compound represented by formula (III-1) or formula (V-1) used in this condensation reaction, it is necessary to deprotect the protecting group of the amino and convert R₅ and R₅' into hydrogen atoms by an appropriate means.

As a means for deprotecting the protecting group of R₅ or converting the phthalimide into an amino, a commonly known means can be employed depending on the protecting group. Specifically, means such as acidic conditions, basic conditions, reduction, nucleophilic reaction, etc. can be employed. Boc, Alloc, a phthalimide, and Cbz can be deprotected under acidic conditions, Fmoc under basic conditions, and Cbz and Alloc under reducing conditions. A phthalimide and Ns can be deprotected by nucleophilic reaction. SES can be deprotected by reaction of fluoride ions. The deprotection of R₅ in the present invention is preferably deprotection under acidic conditions, and more preferably deprotection of Boc under acidic conditions.

When NR₅R₅' represents a nitro in the compound represented by formula (III-1) or formula (V-1) used in this condensation reaction, it is necessary to convert R₅ and R₅' into hydrogen atoms by an appropriate means.

While a commonly known means can be employed for this conversion, it may be, for example, reduction by catalytic hydrogenation using a metal such as Pd, Pt, or Ni with a hydrogen source, reduction using a metal such as Fe, Sn, or Zn with an acid such as ammonium chloride, hydrochloric acid, or acetic acid, reduction using a hydride such as sodium borohydride or lithium aluminum hydride.

According to the present invention, the above condensation reaction can be performed either before or after construction of an acetylene described below. Specifically, formula (III-2) and formula (II) can be derived from formula (III-1) and formula (V-1), respectively. It is preferable to perform this condensation reaction after the construction of the terminal acetylene.

The condensing agent used in the condensation reaction is not particularly limited as long as the reaction proceeds. For example, a combination of a carbodiimide condensing agent, such as 1-(3-dimethylaminopropyl)-3-ethylcarbodiimide or a hydrochloride thereof (WSC or EDC), dicyclohexylcarbodiimide (DCC), 1-cyclohexyl-3-(2-morpholinoethyl)carbodiimide metho-p-toluenesulfonate (CMC), diisopropylcarbodiimide (DIC), and 1,3-bis(2,2-dimethyl-1,3-dioxolan-4-ylmethyl)carbodiimide (BDDC), and an alcohol for constructing an activated ester, such as 1-hydroxybenzotriazole (HOBt), 1-hydroxy-7-azabenzotriazole (HOAt), ethyl(hydroxyimino)cyanoacetate (Oxyma), N-hydroxysuccinimide (HOSu), and pentafluorophenol; an uronium condensing agent such as O-(7-azabenzotriazo-1-yl)-N,N,N',N'-tetramethylhexauronium hexafluorophosphate (HATU) and (1-cyano-2-ethoxy-2-oxoethylideneaminooxy)dimethylamino-morpholinocarbenium hexafluorophosphate (COMU); a 2-halo-N-alkylpyridinium salt (Mukaiyama condensation reagent) such as 2-chloro-6-methyl-1,3-diphenylpyridinium tetrafluoroborate and 1-methyl-2-chloropyridinium iodide; an imidazole condensing agent such as N,N'-carbonyldiimidazole (CDI), and 1,1'-carbonyl-di(1,2,4-triazole) (CDT); a phosphonium condensing agent such as bromotripyrrolidinophosphonium hexafluorophate, chlorotripyrrolidinophosphonium hexafluorophate, 1H-benzotriazol-1-yloxytris(dimethylamino)phosphonium hexafluorophosphate (BOP), (benzotriazol-1-yloxy)tripyrrolidinophosphonium hexafluorophosphate (PyBOP), and (7-azabenzotriazol-1-yloxy)tripyrrolidinophosphonium hexafluorophosphate (PyAOP); a triazine condensing agent such as 4-(4,6-dimethoxy-1,3,5-triazin-2-yl)-4-methylmorpholinium chloride (DMTMM) and 4,6-dimethoxy-1,3,5-triazin-2-yl)-(octoxy-2-oxoethyl)dimethylammonium trifluoromethanesulfonate; 2,4,6-tripropyl-1,3,5,2,4,6-trioxatriphosphorinane-2,4,6-trioxide (T3P); and diphenyl phosphate azide (DPPA), etc. Preferably, it is a combination of a carbodiimide condensing agent and an alcohol for constructing an activated ester, and more preferably a combination of EDC and HOBt. These condensing agents, etc. include polymer-supported reagents.

The solvent used for the condensation reaction is not particularly limited as long as the reaction can proceed. The solvent can be selected from the same solvents used in the step of deprotecting the silicon-linked protecting group. Preferably, the solvent is dichloromethane, chloroform, ethyl acetate, dimethyl sulfoxide, diethyl ether, tetrahydrofuran, 1,4-dioxane, acetone, N,N-dimethylformamide, N-methylpyrrolidone, toluene, acetonitrile, or tetrahydrofuran, more preferably, dichloromethane, chloroform, ethyl acetate, dimethyl sulfoxide, or N,N-dimethylformamide, and still more preferably dichloromethane or chloroform. While the amount of the solvent used is not particularly limited as long as the reaction proceeds, it is preferably 5-100 L, more preferably 15-50 L and still more preferably 25-35 L per 1 kg of the reactant.

In the case where this condensation reaction is performed after constructing the terminal acetylene, the terminal acetylene is preferably protected by a silicon-linked protecting group.

The reaction time of the condensation step is not particularly limited and can be appropriately determined as long as the condensation can be achieved. For example, the reaction time is 1-120 hours, preferably 2-48 hours, more preferably 5-48 hours, preferably 8-36 hours, and more preferably 14-24 hours.

The reaction temperature of the condensation step is not particularly limited and can be appropriately determined as long as the condensation can be achieved. For example, the reaction temperature is 0-60°C, preferably 10-50°C, and more preferably 20-40°C.

Other than the combinational use with the condensing agent represented by formula (VIII) used in the condensation reaction, the compound represented by formula (VIII-1) or the compound represented by formula (VIII-2) may be combined with a base.

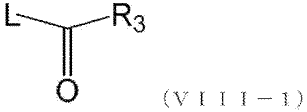

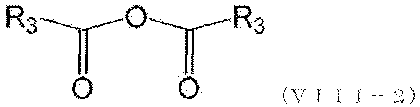

Note that L in the formula represents a halogen atom or a leaving group, and (VIII-1) also includes a mixed acid anhydride, whose definition and embodiment are the same as above and is preferably a halogen atom, and more preferably a chlorine atom.

Examples of the base combined with the compound represented by formula (VIII-1) or the compound represented by (VIII-2) include: organic amines such as trimethylamine, triethylamine, diisopropylethylamine, N-methylmorpholine, diazabicycloundecene (DBU), diazabicyclononene (DBN), pyridine, and 4-dimethylaminopyridine (DMAP); and inorganic basis such as alkali metal hydroxides (lithium hydroxide, sodium hydroxide, potassium hydroxide, etc.), alkaline earth metal hydroxides (barium hydroxide, calcium hydroxide, calcium acetate, etc.), alkali metal carbonates (sodium bicarbonate, sodium carbonate, potassium carbonate, cesium carbonate, etc.), alkaline earth metal carbonates (barium carbonate, calcium carbonate), alkali metal phosphates (sodium phosphate, potassium phosphate, etc.), and acetates (magnesium acetate, potassium acetate, calcium acetate, cesium acetate, etc.).

Next, construction of the terminal acetylene will be described.

Examples of a method for constructing a terminal acetylene generally include Corey-Fuchs method, Seyferth-Gilbert method, Ohira-Bestmann method, and Sonogashira coupling. Corey-Fuchs method requires an aldehyde as a precursor to a dihaloalkene, making it less versatile than Sonogashira coupling which requires an aryl halide, etc. Furthermore, since Corey-Fuchs method requires the use of a strong base, the substrate needs to withstand strong base conditions. Since Seyferth-Gilbert method and Ohira-Bestmann method require the use of explosive 1-diazo-2-oxypropyl phosphonate, there is a concern about mass synthesis of the pharmaceutical ingredients.

Sonogashira coupling, on the other hand, is a cross-coupling reaction between an acetylene derivative and an aryl halide or alkyne halide, and has the advantage that it can be performed without using a strong base or an explosive diazo compound. Moreover, if this acetylene derivative is a compound represented by formula (IV) in which one of the two hydrogen atoms possessed by acetylene has been replaced with a protecting group, it is possible to construct a terminal acetylene represented by general formula (I) or the like by deprotection. Specific examples of the protecting group are as described for R₁.

According to the present invention, Sonogashira coupling can synthesize a compound of formula (V) by reacting a compound represented by formula (III) with a compound represented by formula (IV) below.

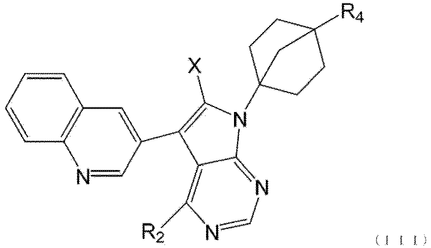

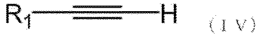

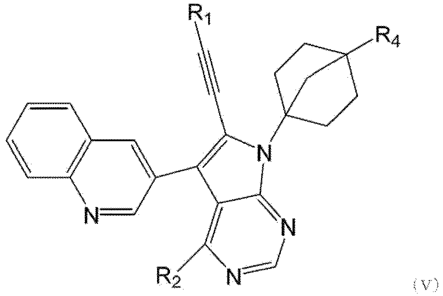

Wherein, in each of the above formulae, X, R₁, and R₂ are as defined above. R₄ represents NHCOR₃ or NR₅R₅' wherein R₅ and R₅' each independently represent a hydrogen atom or a protecting group of an amino, or NR₅R₅' represents a phthalimide or a nitro.

In the case of introducing the compound represented by formula (IV) into the compound represented by formula (III) by Sonogashira coupling, the quantity ratio of the compound represented by formula (III) to the compound represented by formula (IV), the ligand of the catalyst for the reaction, the type and amount of solvent, and the order of addition are also important factors for high purification.

In Sonogashira coupling, the amount of the compound represented by formula (IV) is, for example, 1.0-5.0 moles, preferably 1.25-3.75 moles, and more preferably 1.5-2.5 moles per mole of the compound represented by formula (III).

The catalyst for Sonogashira coupling is not particularly limited as long as it can promote the reaction, but, for example, a palladium catalyst can be used alone or in combination with an activator as necessary.

Examples of the palladium catalyst include, but are not particularly limited to, carbon-supported palladium (Pd-C), alumina-supported palladium, silica gel-immobilized palladium, palladium acetate, dichlorobis[di-tert-butyl(4-dimethylaminophenyl)phosphine]palladium(II), tetrakis(triphenylphosphine)palladium(0), dichloro(1,1-bis(diphenylphosphino)ferrocene)palladium(II), and dichlorobis(triphenylphosphine)palladium(II). Examples of the ligand of the catalyst include, but are not particularly limited to, tri(o-tolyl)phosphine, tri(tert-butyl)phosphine, triphenylphosphine, dichlorobis(triphenylphosphine), 2-di-tert-butylphosphino-2',4',6'-triisopropylbiphenyl, 2-dicyclohexylphosphino-2',6'-dimethoxybiphenyl, [4-(N,N-dimethylamino)phenyl]di-tert-butylphosphine, di-tert-butyl(4-dimethylaminophenyl)phosphine, tricyclohexylphosphine, 4,5-bis(diphenylphosphino)-9,9-dimethylxanthine (Xantphos), and bis(2-(diphenylphosphino)phenyl)ether. The ligand of the catalyst may be one that has been pre-coordinated to the palladium catalyst, or the palladium catalyst and the ligand of the catalyst may separately be placed into a solvent to be coordinated in the reaction solvent.

While examples of the palladium catalyst have been listed above, it is preferably dichlorobis[di-tert-butyl(4-dimethylaminophenyl)phosphine]palladium(II). Moreover, the ligand thereof is preferably di-tert-butyl(4-dimethylaminophenyl)phosphine or tricyclohexylphosphine.

As the activator, a copper catalyst, a silver catalyst, a quaternary ammonium, an aqueous amine solution, or the like can be used. Examples of the copper catalyst include salts with monovalent copper ions, specifically copper(I) halides (copper(I) fluoride, copper(I) chloride, copper(I) bromide, copper(I) iodide, copper(I) trifluoromethanesulfonate, etc.). Examples of the silver catalyst include salts with monovalent silver ions, specifically silver(I) oxide. Examples of the quaternary ammonium include quaternary ammonium hydroxides (tetra-n-butylammonium hydroxide: TBAOH, tetraethylammonium hydroxide: TEAOH, n-hexadecyltrimethylammonium hydroxide, choline hydroxide, etc.). As the aqueous amine solution, an aqueous solution of ammonia, primary amine, secondary amine, tertiary amine, or the like can be used. The activator is preferably a copper catalyst, more preferably a salt with a monovalent copper ion, still more preferably copper(I) bromide, copper(I) iodide, or copper(I) trifluoromethanesulfonate, and particularly preferably copper(I) iodide.

The base used for Sonogashira coupling is not particularly limited as long as the base can result in the introduction of an alkyne, and examples thereof include organic bases such as trimethylamine, diethylamine, diisopropylamine (DIPEA), triethylamine (TEA), N-methylmorpholine, diazabicyclononene (DBN), pyridine, 4-dimethylaminopyridine (DMAP), pyridine, morpholine, quinoline, piperidine, and diazabicycloundecene (DBU), and inorganic bases such as alkali metal hydroxides (lithium hydroxide, sodium hydroxide, potassium hydroxide, etc.), alkaline earth metal hydroxides (barium hydroxide, calcium hydroxide, calcium acetate, etc.), alkali metal carbonates (sodium bicarbonate, sodium carbonate, potassium carbonate, cesium carbonate, etc.), alkaline earth metal carbonates (barium carbonate, calcium carbonate), alkali metal phosphates (sodium phosphate, potassium phosphate, etc.), and acetates (magnesium acetate, potassium acetate, calcium acetate, cesium acetate, etc.). Preferably, the base is an organic base and more preferably diisopropylethylamine.

The solvent used for Sonogashira coupling is not particularly limited as long as it is capable of carrying out the reaction, and examples thereof include N,N-diisopropylethylamine, N-methyl-2-pyrrolidone, dimethyl sulfoxide, N,N-dimethylformamide, N,N-dimethylacetamide, tetrahydrofuran, ethyl acetate, dichloromethane, chloroform, 1,4-dioxane, and toluene. Preferably, the solvent is N-methyl-2-pyrrolidone, N,N-dimethylformamide, or N,N-dimethylacetamide.

The reaction time for Sonogashira coupling is not particularly limited as long as it can result in the introduction of an alkyne and can be set as appropriate. For example, the reaction time is 0.5 to 8 hours, preferably 0.5 to 5 hours, and more preferably 1 to 3 hours.

The reaction temperature for Sonogashira coupling is not particularly limited as long as it can result in the introduction of an alkyne and can be set as appropriate. For example, the reaction temperature is 25-120°C, preferably 50-110°C, and more preferably 80-100°C.

Deprotection of the protecting group of the amino can be performed by a known method according to the protecting group. Such deprotection include deprotection under acidic conditions, deprotection under reducing conditions, deprotection under basic conditions, deprotection with a palladium catalyst, and the like. For example, in the case of Boc, deprotection is generally carried out under acidic conditions, and acidic conditions can be set using any acid as long as the deprotection proceeds. Preferably, the acid is trifluoroacetic acid, hydrochloric acid, or sulfuric acid. Moreover, while the amount of the acid used to set the acidic conditions is not particularly limited as long as the reaction can proceed, it is, for example, preferably 1-100 moles, more preferably 2-50 moles, and still more preferably 3-10 moles per mole of the compound represented by formula (I), formula (III), formula (V) or formula (VI).

The solvent used for the deprotection reaction of the protecting group of the amino is not particularly limited as long as the reaction can proceed. The solvent can be selected from the same solvents used in the step of deprotecting the silicon-linked protecting group. Preferably, the solvent is methanol, ethanol, 2-propanol, acetonitrile, or tetrahydrofuran, more preferably methanol, ethanol, or 2-propanol, and still more preferably methanol. While the amount of the solvent used is not particularly limited as long as the reaction can proceed, it is preferably 1-100 L, more preferably 5-50 L, and still more preferably 10-30 L per 1 kg of the reactant.

By appropriately adjusting the order of deprotection of silicon-linked protecting groups under basic conditions, Sonogashira coupling, conversion into R₃, and deprotection of the protecting group of the amino described above, as well as the amounts of the reagents and solvents, it is possible to produce a highly pure target compound without performing purification by column chromatography.

Compound (1), produced via the above synthesis route, may be amorphous or crystalline, and if crystalline, it may be a single crystal or a mixed crystal of multiple crystals. For crystallization, it is possible to use the synthesized compound (1) without recovering it as a crystal or after recovering it first as a crystal (crude crystal). It is also possible to obtain a desired single crystal by a known purification method. In addition, the crystals herein may be either hydrates or anhydrates.

An embodiment of the present invention also comprises a labeled form of compound (1) or a salt of compound (1), specifically, compound (1), a salt of compound (1), or a compound in which one or more atoms have been replaced with a radioactive or non-radioactive isotope.

Chemical purity as used herein refers to the purity measured by HPLC and therefore when the chemical purity of compound (1) is mentioned, it refers to the purity of compound (1) as measured by HPLC. In this case, the wavelength of the detector used for the purity measurement can be set appropriately. Specifically, the chemical purity of the crystal of compound (1) is preferably 95% or higher, more preferably 98% or higher, and particularly preferably 99% or higher.

### (Avoidance of use of explosive reagents)

In Patent literature 1, an acetylene is constructed by Ohira-Bestmann method to produce compound (1), whereas the method of the present invention does not employ Ohira-Bestmann method.

A commonly known method can be employed for constructing a terminal acetylene without employing Ohira-Bestmann method.

An embodiment of such a method comprises:
reacting a compound represented by formula (VI): 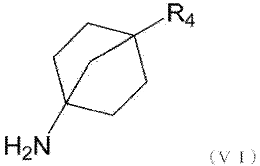 with a compound represented by formula (VII): 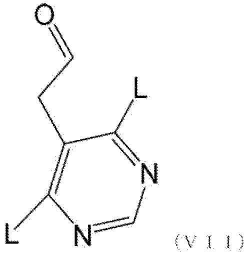
wherein, L represents a halogen atom or a leaving group, to obtain a compound of formula (IX): 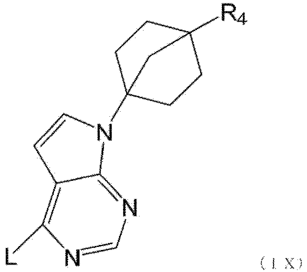
wherein, in formula (VI) and formula (IX),
R₄ represents NR₅R₅',
R₅ and R₅' each independently represent a hydrogen atom or a protecting group of an amino, or NR₅R₅' represents a phthalimide or a nitro, and
L represents a halogen atom or a leaving group; and
obtaining a compound represented by formula (III-1) from the compound of formula (IX). As the method for obtaining the compound represented by formula (III-1) from the compound of formula (IX), for example, the method described in Patent literature 1 can be employed.

### (Purification and other manipulation of crystal)

The precipitated crystal can be isolated and purified from the solution used for dissolving the crystal, the mixed solution, etc. by a known purification technique such as filtration, washing with water or an organic solvent, and drying under reduced pressure.

### (Activities and uses)

The compound or a salt or crystal thereof obtained by the method according to an embodiment of the present invention has excellent EGFR inhibitory activity. In particular, it has excellent inhibitory activity against EGFR (Del19/C797S), EGFR (L858R/C797S), EGFR (Del19/T790M/C797S), and EGFR (L858R/T790M/C797S), and is useful as an antitumor agent. In addition, the compound or a salt or crystal thereof obtained by the method according to an embodiment of the present invention has the advantage of excellent selectivity for mutant EGFRs and fewer side effects caused by wild-type EGFRs and other kinases.

Herein, "wild-type EGFR" is represented, for example, by the amino acid sequence of GenBank accession number: NP_005219.2.

"Exon 19" herein refers to the region 729-823 in the amino acid sequence of wild-type EGFR (e.g., GenBank accession number: NP_005219.2).

"Del19" herein refers to a mutation in which one or more amino acids are deleted in the exon 19 region of wild-type EGFR. A mutation in which any one or more amino acids are inserted in addition to the deletion in the region are also included. Exon 19 deletion mutations include a mutation in which five amino acids from glutamic acid 746 to alanine 750 in the exon 19 region are deleted (Del E746-A750 (also called d746-750)), a mutation in which seven amino acids from leucine 747 to proline 753 in the exon 19 region are deleted, and then followed by insertion of serine (Del L747-P753insS), a mutation in which five amino acids from leucine 747 to threonine 751 in the exon 19 region are deleted (Del L747-T751), and a mutation in which 4 amino acids from leucine 747 to alanine 750 in the exon 19 region are deleted, and then followed by insertion of proline (Del L747-A750insP), etc. Preferably, it includes the mutation in which five amino acids from glutamic acid 746 to alanine 750 in the exon 19 region are deleted (Del E746-A750).

The compound or a salt thereof obtained by the method according to an embodiment of the present invention may be used in postoperative adjuvant chemotherapy that is given after surgical resection of a tumor to prevent recurrence, or in preoperative adjuvant chemotherapy given before surgical resection of a tumor.

While the tumor targeted by the present invention is not particularly limited, examples thereof include head and neck cancer, gastrointestinal cancer (esophageal cancer, stomach cancer, duodenal cancer, liver cancer, biliary tract cancer (gallbladder cancer, bile duct cancer, etc.), pancreatic cancer, large bowel cancer (colorectal cancer, colon cancer, rectal cancer, anal cancer, etc.), etc.), lung cancer (non-small cell lung cancer, small cell lung cancer, mesothelioma (pleural mesothelioma, peritoneal mesothelioma, pericardial mesothelioma, testicular mesothelioma, etc.)), breast cancer, genital cancer (ovarian cancer, vulvar cancer, uterine cancer (uterine cervical cancer, uterine body cancer, endometrial cancer, etc.), etc.), urogenital cancer (renal cancer, bladder cancer, prostate cancer, testicular tumor, urothelial cancer, renal pelvis cancer, urethral cancer, etc.), hematopoietic tumor (leukemia, malignant lymphoma, multiple myeloma, etc.), bone and soft tissue tumors, rhabdomyosarcoma, skin cancer, brain tumor, malignant schwannoma, neuroendocrine tumor, and thyroid cancer. Preferably, it is head and neck cancer, breast cancer, large bowel cancer, esophageal cancer, pancreatic cancer, lung cancer, ovarian cancer, renal cancer, bladder cancer, skin cancer, or brain tumor, and particularly preferably it is lung cancer. Here, cancers include not only their primary foci but also cancers that have metastasized to other organs (e.g., liver). Furthermore, the compound or a salt or crystal thereof obtained by the method according to an embodiment of the present invention has excellent EGFR inhibitory activity. Examples of such mutant EGFR include drug-resistant mutant EGFRs and highly-sensitive mutant EGFRs. Therefore, the compound or a salt thereof of the present invention is useful as an antitumor agent against the aforementioned malignant tumors with mutant EGFR.

As used herein, the term "effective amount" of the compound refers to the amount (therapeutically effective amount) of the compound of the present invention, which can cause a biological or medical response such as reduction or inhibition of enzyme and/or protein activity in a subject or which can ameliorate symptoms, alleviate conditions, slow or delay the progress of a disease.

As used herein, the term "subject" encompasses mammals and non-mammals. In an embodiment, the subject is a human, and may be a human who has been diagnosed as being in need of a treatment for a symptom, condition, or disease disclosed herein.

A crystal or co-crystal of the compound (1) or a salt thereof produced by the method described above can be used in any various dosage form depending on the purpose of treatment, with or without pulverizing the crystal, and can be used in a formulation commonly used for pharmaceutical purposes. The dosage form may be, for example, any of oral dosage forms such as tablets, capsules, granules, fine granules, dispersions, and dry syrups; and parenteral dosage forms such as suppositories, inhalants, nasal sprays, ointments, plasters, and injections. Pharmaceutical compositions suitable for these dosage forms can be produced by formulation methods known and customary to those skilled in the art, using pharmacologically acceptable carriers.

Various organic and inorganic carrier substances customarily used as formulation ingredients can be used as the pharmacologically acceptable carrier, where examples thereof that can be incorporated include an excipient, a binder, a disintegrant, a lubricant, and a coating agent in solid formulations, and a solvent, a solubilizer, a suspending agent, an isotonic agent, a buffering agent, and a soothing agent in liquid formulations. In addition, an antiseptic, an antioxidant, a coloring agent, a sweetener, a stabilizer, and other formulation additive may be used if necessary.

In the case of preparing an oral solid formulation, the compound (1) and a crystal thereof produced by the method described above (i.e., the crystal of compound (1) in free form or the crystal of compound (1) with an acid (crystal of salt or co-crystal)) may be mixed with an excipient and optionally with a binder, a disintegrant, a lubricant, a coloring agent, a flavoring agent, etc., and then formulated into tablets, coated tablets, granules, powders, capsules, etc. by a conventional method.

In the case of preparing an injection, the compound (1) and a crystal thereof produced by the method described above (i.e., the crystal of compound (1) in free form or the crystal of compound (1) with an acid (crystal of salt or co-crystal)) may be mixed with a pH adjuster, a buffering agent, a stabilizer, an isotonic agent, a local anesthetic, etc., and then formulated into injections for subcutaneous, intramuscular, and intravenous use by a conventional method.

The amount of the compound (1) and a crystal thereof produced by the method described above (i.e., the crystal of compound (1) in free form or the crystal of compound (1) with an acid (crystal of salt or co-crystal)) to be incorporated into each dosage unit form will vary depending on the symptom of the patient to whom it is applied, or on the formulation thereof. In general, the amount per dosage unit form in terms of compound (1) in free form is desirably about 0.05-1,000 mg for oral formulations, about 0.1-500 mg for injections, and about 1-1,000 mg for suppositories or topical formulations.

Moreover, the daily dose of compound (1) and a crystal thereof produced by the method described above (i.e., compound (1) produced by the method described above, the crystal of compound (1) in free form, or the crystal of compound (1) with an acid (crystal of salt or co-crystal)) of a drug in each dosage form will vary depending on the symptom, body weight, age, sex, etc. of the patient. In general, the daily dose for adults (body weight: 50 kg) in terms of compound (1) in free form may be about 0.05 to 5,000 mg and preferably 0.1 to 1,000 mg.

### (Exemplary embodiments)

Hereinafter, exemplary embodiments of the present invention will be described, but the present invention should not be limited to the following embodiments.

While a method according to an embodiment of the present invention relates to a method for producing a compound represented by formula (I) or a salt thereof by deprotecting a protecting group represented by R₁ of a compound represented by formula (II), it is preferably a method for producing compound (1) or a salt thereof by deprotecting a protecting group represented by R₁ in formula (II) under basic conditions, more preferably said method which is performed under basic conditions adjusted by a reagent containing no fluoride ion, and still more preferably said method wherein R₁ is triethylsilyl.

A method in another embodiment of the present invention is a method for producing a compound represented by formula (I), the method comprising the steps performed in the order (A)-(B) below.
(A) Step of reacting a compound represented by formula (III-2) with a compound represented by formula (IV) to produce a compound represented by formula (II).
(B) Step of converting the compound represented by formula (II) into a compound represented by formula (I).

A method in another embodiment of the present invention is a method for producing a compound represented by formula (I) or a salt thereof, the method comprising (A)-(C) below:
(A) linking a compound represented by formula (III-1) with a compound represented by formula (VIII) to give a compound represented by formula (III-2), or linking a compound represented by formula (III-1) with a compound represented by formula (IV) to produce a compound represented by formula (V-1);
(B) linking the compound represented by formula (III-2) with a compound represented by formula (IV) or linking the compound represented by formula (V-1) with a compound represented by formula (VIII) to give a compound represented by formula (II); and
(C) deprotecting a protecting group represented by R₁ in formula (II) under basic conditions to form a compound represented by formula (I) or a salt thereof.

It is preferably a method for producing a compound represented by formula (I) or a salt thereof, the method comprising (A)-(C) below:
(A) reacting a compound represented by formula (III-1) with a compound represented by formula (IV) to produce a compound represented by formula (V-1);
(B) converting the compound represented by formula (V-1) into a compound represented by formula (II); and
(C) converting the compound represented by formula (II) into a compound represented by formula (I) under basic conditions.

More preferably, it is a method for producing a compound represented by formula (I) or a salt thereof, wherein R₁ is a silicon-linked protecting group, R₂ is an amino, R₃ is a pyrazinyl optionally having a methyl group, R₄ is NR₅R₅', R₅ is a protecting group which can be deprotected under acidic conditions, R₅' is a hydrogen atom, X is a halogen atom, and L is a halogen atom, and the content of a compound represented by formula (I-1) is 0.2% or less, the method comprising (A)-(C) below but not comprising a step of purification by column chromatography:
(A) reacting a compound represented by formula (III-1) with a compound represented by formula (IV) to produce a compound represented by formula (V-1);
(B) deprotecting a protecting group, which can be deprotected under acidic conditions, of the compound represented by formula (V-1) and condensing the compound with a compound represented by formula (VIII) to give a compound represented by formula (II); and
(C) converting the compound represented by formula (II) into a compound represented by formula (I) under basic conditions.

Note that the compound represented by formula (III-1) is derived from a compound of formula (IX), which is obtained by reacting a compound represented by formula (VI) with a compound represented by formula (VII).

Still more preferably, it is a method for producing compound (1) or a salt thereof, wherein R₁ is a silicon-linked protecting group, R₂ is an amino, R₃ is 5-methylpyrazin-2-yl, R₄ is NR₅R₅', R₅ is a protecting group which can be deprotected under acidic conditions, R₅' is a hydrogen atom, X is a halogen atom, and L is a halogen atom, and the content of compound (2) is 0.2% or less, the method comprising (A)-(C) below but not comprising a step of purification by column chromatography:
(A) reacting a compound represented by formula (III-1) with a compound represented by formula (IV) to produce a compound represented by formula (V-1);
(B) deprotecting a protecting group of the compound represented by formula (V-1), which can be deprotected under acidic conditions, and condensing the compound with 5-methylpyrazine-2-carboxylic acid to give a compound represented by formula (II); and
(C) converting the compound represented by formula (II) into compound (1) under basic conditions.

Note that the compound represented by formula (III-1) is derived from a compound of formula (IX), which is obtained by reacting a compound represented by formula (VI) with a compound represented by formula (VII).

Yet still more preferably, it is a method for producing compound (1) or a salt thereof, wherein R₁ is TES, R₂ is an amino, R₃ is 5-methylpyrazin-2-yl, R₄ is NR₅R₅', R₅ is Boc, R₅' is a hydrogen atom, X is a bromine atom, and L is a chlorine atom, and the content of compound (2) is 0.2% or less, the method comprising (A)-(C) below and but not comprising a step of purification by column chromatography:
(A) reacting a compound represented by formula (III-1) with a compound represented by formula (IV) using a palladium catalyst and a copper catalyst to produce a compound represented by formula (V-1);
(B) deprotecting Boc of the compound represented by formula (V-1) and condensing the compound with 5-methylpyrazine-2-carboxylic acid to give a compound represented by formula (II); and
(C) converting the compound represented by formula (II) into compound (1) under basic conditions adjusted by a reagent containing no fluoride ion.

Note that the compound represented by formula (III-1) is derived from a compound of formula (IX), which is obtained by reacting a compound represented by formula (VI) with a compound represented by formula (VII).

Particularly preferably, it is a method for producing compound (1) or a salt thereof, wherein R₁ is TES, R₂ is an amino, R₃ is 5-methylpyrazin-2-yl, R₄ is NR₅R₅', R₅ is Boc, R₅' is a hydrogen atom, X is a bromine atom, and L represents a chlorine atom, and the content of compound (2) is 0.2% or less, the method comprising the steps (A)-(C) below but not comprising a step of purification by column chromatography.
(A) Step of reacting a compound represented by formula (III-1) with a compound represented by formula (IV) using dichlorobis[di-tert-butyl(4-dimethylaminophenyl)phosphine]palladium(II) and copper(I) iodide to produce a compound represented by formula (V-1).
(B) Step of deprotecting Boc of the compound represented by formula (V-1) under acidic conditions and condensing the compound with 5-methylpyrazine-2-carboxylic acid using a combination of EDC and HOBt to give a compound represented by formula (II).
(C) Step of converting the compound represented by formula (II) into a compound represented by formula (I) under basic conditions adjusted by potassium
carbonate.

Note that the compound represented by formula (III-1) is derived from a compound of formula (IX), which is obtained by reacting a compound represented by formula (VI) with a compound represented by formula (VII).

A method in another embodiment of the present invention is a method for producing a composition comprising a compound represented by formula (I) or a salt thereof and a compound represented by formula (I-1). This method is a method in which the percentage of the compound represented by formula (I-1) is 0.2% or less, the method comprising the steps performed in the order (A)-(C) below but not comprising a step of purification by column chromatography.
(A) Step of reacting a compound represented by formula (III-1) with a compound represented by formula (IV) to produce a compound represented by formula (V-1).
(B) Step of converting the compound represented by formula (V-1) into a compound represented by formula (II).
(C) Step of converting the compound represented by formula (II) into a compound represented by formula (I) under basic conditions.

A method in another embodiment of the present invention is a method for producing a composition comprising a compound represented by formula (I) or a salt thereof and a compound represented by formula (I-1). This method is a method in which the percentage of the compound represented by formula (I-1) is 0.2% or less, the method comprising the steps performed in the order (A)-(C) below but not comprising a step of purification by column chromatography.
(A) Step of converting a compound represented by formula (III-1) into a compound represented by formula (III-2).
(B) Step of converting the compound represented by formula (III-2) into a compound represented by formula (II).
(C) Step of converting the compound represented by formula (II) into a compound represented by formula (I) under basic conditions.

### EXAMPLES

Hereinafter, the present invention will be further described in detail by means of examples, but the invention should not be limited in any way to these examples. Although the invention is fully described by the examples, it is understood that various changes and/or modifications may be made by those skilled in the art. Therefore, as long as such changes and/or modifications do not depart from the scope of the invention, they are included in the present invention.

In the following examples of compounds, % indicates weight percent unless otherwise noted.

The equipment, including data processing, was handled in accordance with the method and procedure indicated for the equipment.

Note that the values obtained from various spectra may somewhat vary depending on the measurement conditions and the like. Therefore, these values should not be strictly interpreted.
Equipment: UPLC provided by Waters (% indicates percent volume)
Column: ACQUITY UPLC BEH C18 Column, 130Å, 1.7 µm, 2.1 mm x 50 mm (Waters)
Column temperature: 40°C
Measurement wavelength: 254 nm
Flow rate: 0.5 mL/min.
Mobile phase A: 0.1% aqueous formic acid solution
Mobile phase B: 0.1% formic acid-acetonitrile
Injection volume: 1 µL
Gradient: from 0 to 0.1 min: Mobile phase B 5%, from 0.1 to 2.1 min: Mobile phase B from 5 to 95%, from 2.1 to 3.5 min: Mobile phase B from 95 to 98%, from 3.5 to 5 min: Mobile phase B from 98 to 95%

The equipment, including data processing, was handled in accordance with the method and procedure indicated for the equipment.

### Example 1: Production of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (compound (1))

### (Step 1) Synthesis of tert-butyl(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)carbamate

To tert-butyl(4-(4-amino-6-bromo-5-(quinolin-3-yl)-7H-pyrrolo[2,3-d]pyrimidin-7-yl)bicyclo[2.2.1]heptan-1-yl)carbamate (65.9 g), which was synthesized according to the method described in Patent literature 1, N-methylpyrrolidone (1500 mL) was added and stirred at 90°C for 30 minutes in a nitrogen atmosphere. After cooling to 35°C, diisopropylethylamine (46.5 g), triethylsilylacetylene (33.7 g), copper(I) iodide (4.57 g), dichlorobis[di-tert-butyl(4-dimethylaminophenyl)phosphine]palladium(II) (8.50 g), and N-methylpyrrolidone (80 mL) were added and the mixture was stirred at 90°C for 2 hours in a nitrogen atmosphere. After cooling to 40°C, 10% aqueous sodium dihydrogen phosphate solution and ethyl acetate were added. The organic layer was washed with diluted ammonia and water, to which activated carbon (33.0 g) and SH silica gel (65.9 g) were added and stirred at 40°C for 1 hour and at room temperature for 2 hours. The insoluble matter was filtered over Celite, then the solvent was removed under reduced pressure, and acetonitrile was added to the resulting residue and stirred at room temperature for 15 hours. The precipitated solid was filtered off, washed with acetonitrile, and dried under reduced pressure to give the title compound (37.4 g, 51% yield).

### (Step 2) Synthesis of 7-(4-aminobicyclo[2.2.1]heptan-1-yl)-5-(quinolin-3-yl)-

### 6-((triethylsilyl)ethynyl)-7H-pyrrolo[2,3-d]pyrimidin-4-amine

To tert-butyl(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)carbamate (65.0 g), which was synthesized as described above, 5-10% hydrochloric acid methanol solution (520 mL) was added and stirred at 50°C for 3 hours in a nitrogen atmosphere. Under ice-cold conditions, 2 mol/L aqueous sodium hydroxide solution was added dropwise to adjust the pH to around 12. The precipitated solid was filtered off, washed with 50% methanol, and dried under reduced pressure to give the title compound (50.3 g, 93% yield).

### (Step 3) Synthesis of N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide

1-(3-dimethylaminopropyl)-3-ethylcarbodiimide hydrochloride (21.9 g) was added to a mixture of 7-(4-aminobicyclo[2.2.1]heptan-1-yl)-5-(quinolin-3-yl)-6-((triethylsilyl)ethynyl)-7H-pyrrolo[2,3-d]pyrimidin-4-amine obtained in step 2 (48.3 g), 5-methylpyrazine-2-carboxylic acid (13.4 g), 1-hydroxybenzotriazole monohydrate (16.0 g), diisopropylethylamine (24.6 g), and dichloromethane (1,450 mL) in a nitrogen atmosphere and stirred for 17 hours. The reaction solution was washed with 10% aqueous sodium dihydrogen phosphate solution, a saturated aqueous sodium carbonate solution, and saturated saline, and dried with anhydrous sodium sulfate. The drying agent was filtered off, then the solvent was removed under reduced pressure, and methanol was added to the resulting residue and stirred at room temperature for 2 hours. The precipitated solid was filtered off, washed with methanol, and dried under reduced pressure to give the title compound (54.8 g, 92% yield).

### (Step 4) Synthesis of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (compound (1))

Potassium carbonate (7.03 g) was added to a mixture of N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide obtained in step 3 (80.0 g), dichloromethane (1,000 mL), and methanol (1,000 mL) in a nitrogen atmosphere and stirred for 18 hours. The insoluble matter was filtered over Celite, then the solvent was removed under reduced pressure, and methanol and water were added to the resulting residue and stirred for 1 hour. The precipitated solid was filtered off, washed with 50% methanol, and dried under reduced pressure to give crystal of the title compound (64.5 g, 99% yield) as a pale yellow solid.

The compound obtained was analyzed by ¹H-NMR, which gave the same results as in Example 37 of Patent literature 1.

In addition, UPLC analysis showed that the purity was 99.73% and the content of N-(4-(6-ethynyl-4-(5-methylpyrazine-2-carboxamide)-5-(quinolin-3-yl)-7H-pyrrolo[2,3-d]pyrimidin-7-yl)bicyclo[2.2.1]heptan-1-yl)-5-methylpyrazine-2-carboxamide was below the detection limit (<0.05%).

### Comparative example 1: Synthesis of compound (1) by method comprising condensation as the final step

According to the description in Example 37 of Patent literature 1, 7-(4-aminobicyclo[2.2.1]heptan-1-yl)-6-ethynyl-5-(quinolin-3-yl)-7H-pyrrolo[2,3-d]pyrimidin-4-amine (1 equivalent) obtained in Production example 1 of Patent literature 1, 5-methylpyrazine-2-carboxylic acid (1 equivalent), and diisopropylethylamine (1.5 equivalents) were added to dimethyl sulfoxide, which was in an amount of 20 mL per 1 g of the compound obtained in Production example 1 of Patent literature 1, and HATU (1.2 equivalents) was added to the mixture and stirred at room temperature for 1.5 hours. After confirming the completion of the reaction, water, which was about 1/3 volume of the above dimethyl sulfoxide, was added and the precipitate obtained was filtered off and dried to give compound (1) in a yield of 93%. UPLC analysis of the obtained compound (1) showed that the purity was 97.3% but 1.42% N-(4-(6-ethynyl-4-(5-methylpyrazine-2-carboxamide)-5-(quinolin-3-yl)-7H-pyrrolo[2,3-d]pyrimidin-7-yl)bicyclo[2.2.1]heptan-1-yl)-5-methylpyrazine-2-carboxamide was contained.

All references and publications mentioned herein are incorporated herein by reference in their entirety, regardless of purpose. This specification also includes the disclosure of the claims, description, and drawings of Japanese patent application No. 2022-071155 (filed on April 22, 2022), to which priority is claimed by this application.

Some embodiments of the present invention have been described but these embodiments are presented as examples and are not intended to limit the scope of the invention. These novel embodiments can be implemented in various other embodiments, and various omissions, replacements, and modifications can be made without departing from the gist of the invention. These embodiments and variations thereof are included in the scope and gist of the invention as well as in the scope of the invention and its equivalents recited in the claims.

## Claims

1. A method for producing a compound represented by formula (I) or a salt thereof, the method comprising deprotecting a protecting group represented by R₁ in formula (II): 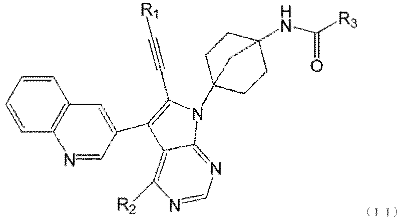 under basic conditions to form a compound represented by formula (I) or a salt thereof: 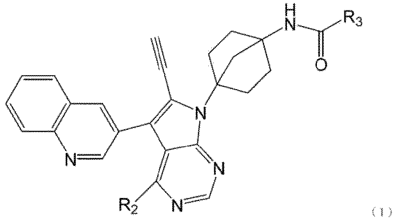
wherein, in formulae (I) and (II) above,
R₁ represents a protecting group,
R₂ represents an optionally protected amino, a halogen atom, or a leaving group, and
R₃ represents an optionally substituted 5-10 membered monocyclic or polycyclic unsaturated heterocyclic ring group.

2. The method according to claim 1, wherein, in formulae (I) and (II), R₁ represents a silicon-linked protecting group, R₂ represents an amino, and R₃ represents a pyrazinyl optionally having a methyl group.

3. The method according to claim 1 or 2, wherein the compound represented by formula (I) is N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof.

4. A method for producing a compound represented by formula (I) or a salt thereof, the method comprising:
(1) linking a compound represented by formula (III-1) with a compound represented by formula (VIII) to give a compound represented by formula (III-2), or linking a compound represented by formula (III-1) with a compound represented by formula (IV) to produce a compound represented by formula (V-1): 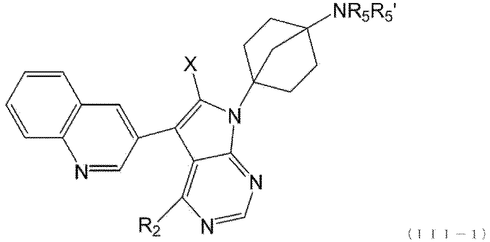 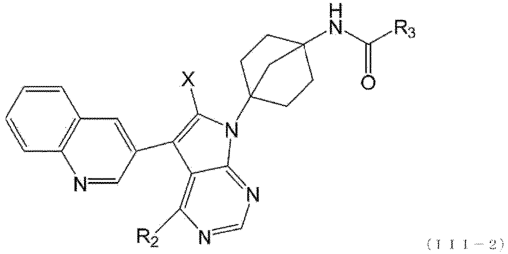 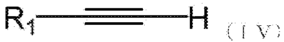 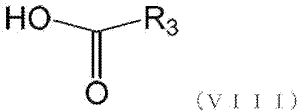 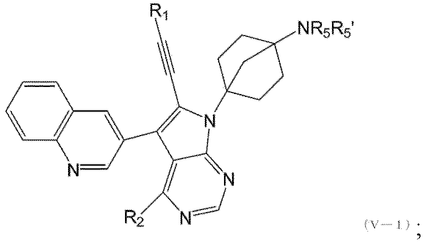
(2) linking the compound represented by formula (III-2) with the compound represented by formula (IV) or linking the compound represented by formula (V-1) with the compound represented by formula (VIII) to give a compound represented by formula (II); and 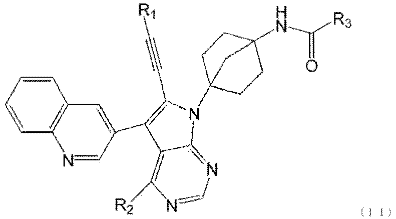
(3) deprotecting a protecting group represented by R₁ in formula (II) under basic conditions to form the compound represented by formula (I) or a salt thereof: 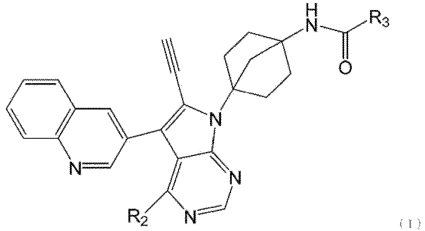
wherein, in formulae (I), (II), (III-1), (III-2), (IV), (V-1), and (VIII) above,
R₁ represents a protecting group,
R₂ represents an optionally protected amino, a halogen atom, or a leaving group,
R₃ represents an optionally substituted 5-10 membered monocyclic or polycyclic unsaturated heterocyclic ring group,
R₅ and R₅' each independently represent a hydrogen atom or a protecting group of an amino, or NR₅R₅' represents a phthalimide or a nitro, and
X represents a halogen atom or a leaving group.

5. The method according to claim 4, wherein in formulae (I), (II), (III-1), (III-2), (IV), and (V-1) above,
R₁ represents a silicon-linked protecting group,
R₂ represents an amino,
R₃ represents a pyrazinyl optionally having a methyl group,
R₄ represents NR₅R₅', and
R₅ represents a hydrogen atom or Boc, and R₅' represents a hydrogen atom.

6. The method according to claim 4 or 5, wherein the compound represented by formula (I) is N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof.

7. The method according to claim 4, further comprising:
reacting a compound represented by formula (VI): 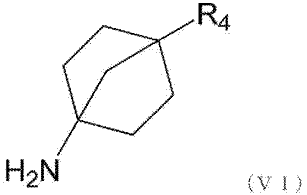 with a compound represented by formula (VII): 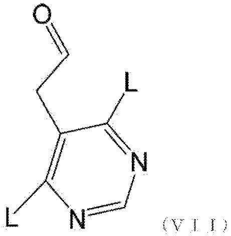
wherein, L represents a halogen atom or a leaving group, to obtain a compound of formula (IX): 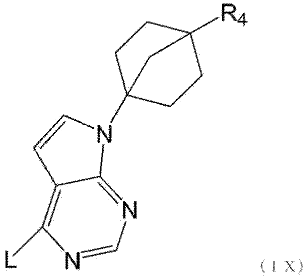
wherein, in formula (VI) and formula (IX),
R₄ represents NR₅R₅',
R₅ and R₅' each independently represent a hydrogen atom or a protecting group of an amino, or NR₅R₅' represents a phthalimide or a nitro, and
L represents a halogen atom or a leaving group; and
obtaining a compound represented by formula (III-1) from the compound of formula (IX).

8. The method according to claim 7, wherein, in formulae (VI), (VII), and (IX) above,
R₄ represents NR₅R₅',
R₅ represents Boc, and R₅' represents a hydrogen atom, and
L represents a chlorine atom.

9. The method according to claim 4, wherein the method does not comprise purification by column chromatography.

10. The method according to claim 9, wherein the content of a compound represented by formula (I-1): 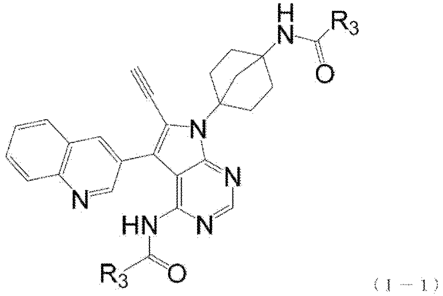 wherein, R₃ represents an optionally substituted 5-10 membered monocyclic or polycyclic unsaturated heterocyclic ring group,
in the compound represented by formula (I) and a salt thereof produced by the method is 0.2% or less.

11. A composition obtained by the method according to claim 10, comprising the compound represented by formula (I) or a salt thereof.

12. A compound represented by formula (II) or a salt thereof: 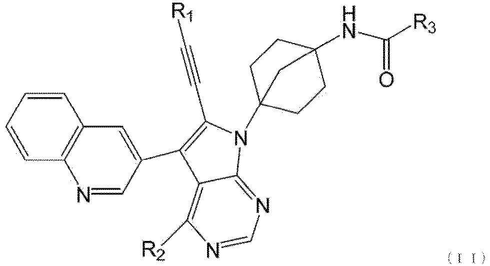 wherein,
R₁ represents a protecting group,
R₂ represents an optionally protected amino, a halogen atom, or a leaving group, and
R₃ represents an optionally substituted 5-10 membered monocyclic or polycyclic unsaturated heterocyclic ring group.

13. The compound or a salt thereof according to claim 12, wherein, in formula (II), R₁ represents a silicon-linked protecting group, R₂ represents an amino, and R₃ represents a pyrazinyl optionally having a methyl group.

14. The compound or a salt thereof according to claim 12 or 13, wherein the compound represented by formula (II) is N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide or a salt thereof.
